# EUROPEAN PATENT SPECIFICATION

(11) **EP 0 269 239 B1**
(45) Date of publication and mention of the grant of the patent: **08.06.1994**
(21) Application number: 87309159.9
(22) Date of filing: 16.10.1987
(51) Int. Cl.: C07D 417/04, C07C 335/28

(54) **Processes for 2-(1-pentyl-3-guanidino)-4-(2-methyl-4-imidazolyl)thiazole and the crystalline dihydrochloride trihydrate thereof**
Verfahren zur Herstellung von 2-(1-Pentyl-3-guanidino)-4-(2-methyl-4-imidazolyl)Thiazol und dessen kristallinisches Dihydrochlorid-Trihydrat
Procédés de préparation de (pentyl-1-guanidino-3)-2-(méthyl-2-imidazolyl-4)-4-thiazole et son dihydrochloride trihydrate cristallin

(30) Priority: 29.10.1986 WO PCT/US86/02307; 29.10.1986 WO PCT/US86/02308
(43) Date of publication of application: 01.06.1988
(73) Proprietor: PFIZER INC., New York, N.Y. 10017 (US)
(72) Inventor: Allen, Douglas John Meldrum, Ledyard Connecticut (US); Snyder, William Michael, New London Connecticut (US); Cue, Berkeley Wendell, Jr., Gales Ferry Connecticut (US); Walinsky, Stanley Walter, Mystic Connecticut (US); Hill, Paul David, Groton Connecticut (US)
(74) Representative: Wood, David John

(56) References cited:
- EP-A- 0 050 458
- EP-A- 0 094 191
- EP-A- 0 161 841
- US-A- 391 473
- US-A- 3 798 269
- CHEMICAL ABSTRACTS, vol. 81, no. 3, 22nd July 1974, page 296, abstract no.13068a, Columbus, Ohio, US; C.P. JOSHUA et al.: "Synthesis ofamidinothioureas"

## Description

The present invention is directed to advantageous processes for the preparation of 2-(1-pentyl-3-guanidino)-4-(2-methyl-4-imidazolyl)thiazole, and to a crystalline, hydrated dihydrochloride salt thereof having advantageous properties.

Reiter, (US-A-4,560,690 corresponding to EP-A-0161841) has described 2-(1-pentyl-3-guanidino)-4-(2-methyl-4-imidazolyl)thiazole and various analogs, compounds having valuable antiulcer activity. That patent, further details pharmaceutical compositions and a method of inhibiting ulcers with these compounds.

Reiter prepared the subject compounds via the following reaction scheme: 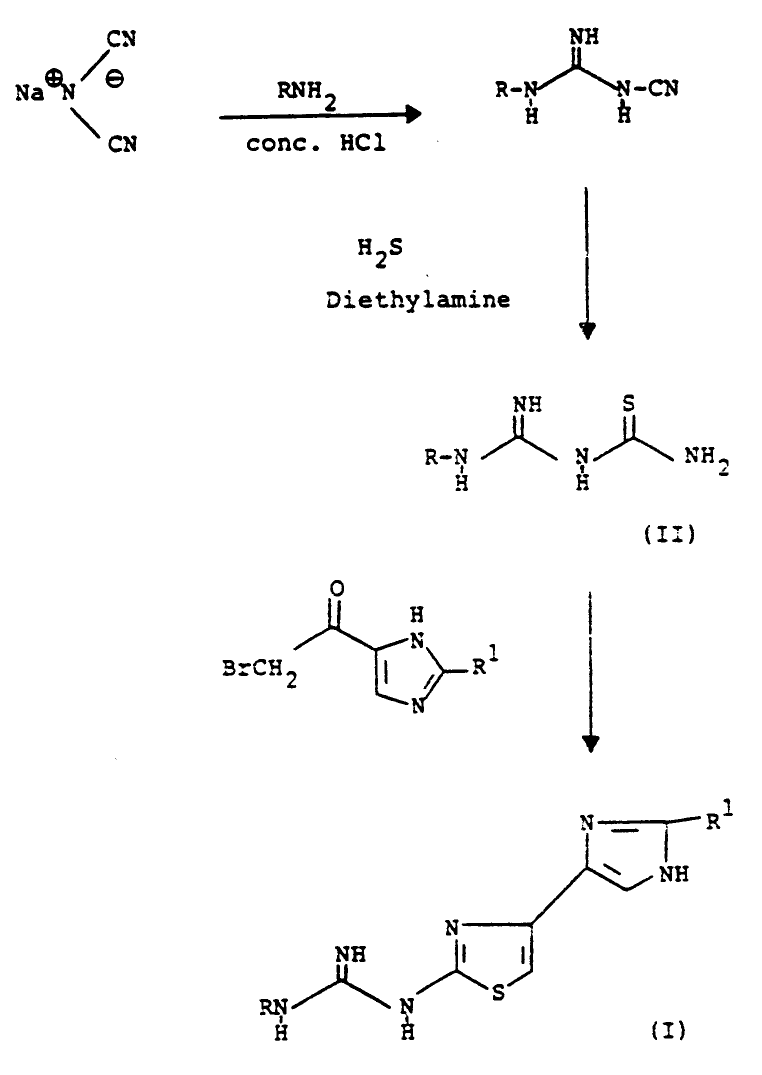
wherein R is benzyl or (C₁-C₅)alkyl and R¹ is (C₁-C₅)alkyl. The products were generally isolated as their dihydrobromide salts and converted to preferred dihydrochloride salts via their free base forms. This route employs toxic reagents, sodium dicyanamide and hydrogen sulfide, presenting environmental problems. In particular, the use of large excesses of hydrogen sulfide (or alternatively hydrogen sulfide under pressure) presents particular problems which limit batch sizes. This old scheme further lacks flexibility, it being necessary to carry out the entire sequence for each compound of the formula (I) that is desired.

EP-A-0050458 refers to 2-guanidino-4-heteroarylthiazoles which are unsubstituted at the guanidino group. Chem. Abs. 81 (1974), 13068a and US-A-3798269 disclose amidinothioureas and their preparation from 1-subst-3-cyano-guanidines.

The present invention is directed to highly flexible routes to the compound of the formula (I) in which R¹ is methyl and R is n-pentyl, which avoid toxic sodium dicyanimide and hydrogen sulfide, and employ an unprecedented acid catalyzed amine exchange reaction, carried out by the action of n-pentylamine either on 2-guanidino-4-(2-methyl-4-imidazolyl)-thiazole, of the formula 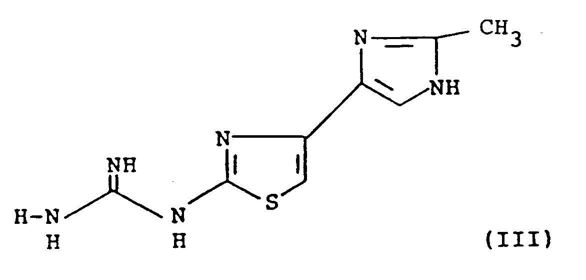
directly forming the compound of the formula (I), as defined above; or on an amidinothiourea (IV) 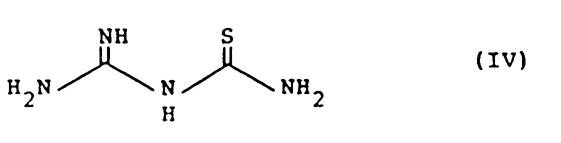
to form the N-substituted aminothiourea of the formula (II) as defined above in which R is n-pentyl; neat or in the presence of a reaction inert solvent.

In one of the preferred embodiments of the present invention, an acid salt of (III), e.g., the dihydrochloride, is reacted with at least one molar equivalent of the amine neat or in the presence of a reaction-inert solvent or solvents (preferably in an excess of the amine) to directly form the corresponding acid salt of (I).

In a second preferred embodiment of the present invention, amidinothiourea (IV) is reacted with at least one molar equivalent of n-pentylamine in a reaction-inert solvent such as a lower alkanol in the presence of a molar excess of an acid, conveniently acetic acid.

The expression "reaction-inert solvent" refers to a solvent which does not interact with starting materials, intermediates or products in a manner which adversely affects the yield of the desired product.

The present invention is also directed to crystalline 2-(1-pentyl-3-guanidino)-(2-methyl-4-imidazolyl)-thiazole dihydrochloride trihydrate, having distinct advantages over the prior anhydrous dihydrochloride of Reiter, which is amorphous, less readily purified, and possesses properties which are generally less suitable for formulation and use as a medicinal agent in man.

The processes of the present invention are readily carried out. When the product is directly the guanylthiazole derivative (I), the reaction is conveniently carried out employing an isolated mono or diacid salt of the compound (III), thus providing the source of the required acid catalyst. The acid catalyst can be a strong acid (e.g. HBr, HCl, pCH₃C₆H₄SO₃H) or a weak acid (e.g. CH₃COOH, NH₄Cl). It is most preferred to use the dihydrochloride salt of (III). The salt is heated with at least one molar equivalent of n-pentylamine. The temperature, which is generally well above ambient temperature, is not a critical feature of the present invention, temperatures in the range of 50-150°C being generally satisfactory, the preferred range being 95-115°C. At temperatures which are above the boiling point of the amine, it will be necessary to carry out the reaction under pressure. The reaction is optionally carried out in the presence of a reaction-inert solvent as diluent, but it is preferred to simply carry out the reaction neat, in the presence of an excess of the amine (e.g. 5-20 molar equivalents).

The starting compounds (III), are conveniently prepared according to LaMattina et al. US-A-4,374,843. LaMattina et al. reported isolation of the compound (III) as its monohydrobromide salt. The latter is better prepared as its dihydrobromide salt according to Cue, EP-A-178,123, published April, 1986. The latter is readily converted via its free base form to other acid salts, such as the presently preferred dihydrochloride salt.

When the product of the present process is the intermediate N-substituted guanylthiourea derivative (II), the amine exchange reaction is carried out under substantially identical conditions, although preferably at temperatures lower in the generally satisfactory range noted above (e.g. 50-90°C), conveniently on the commerically available "free base" form of the guanylurea (IV) in the presence of at least one molar equivalent each of n-pentylamine and of the acid (conveniently acetic acid), preferably in the presence of a reaction inert solvent, such as ethanol, as diluent.

The present amine exchange reactions are conveniently monitored by standard methods of TLC (thin layer chromatography) and HPLC (high performance liquid chromatography). The intermediates (II) are converted to the guanylthiazole derivatives according to the methods of Reiter, schematically represented above.

The present crystalline 2-(1-pentyl-3-guanidino)-4-(2-methyl-4-imidazolyl)thiazole dihydrochloride trihydrate is obtained by crystallization from dilute aqueous hydrochloric acid, optionally diluted with a water miscible organic solvent such as acetonitrile, at temperatures generally in the range of 0-50°C. If desired, it is recrystallized from aqueous solvents (e.g., water/acetonitrile) in the same temperature range. At temperatures below 50°C and ambient pressures, the trihydrate is stable, remaining in the crystalline trihydrate form. At tempertures above 50°C (e.g. at 70°C and ambient pressure) it slowly loses the water of hydration, but will readily regain water on storage at ambient temperature and high relative humidity. The trihydrate shows a characteristic strong endotherm at 107-109°C in differential scanning calorimetry.

The following examples are given by way of illustration

### EXAMPLE 1

### 2-(1-Pentyl-3-guanidino)-4-(2-methyl-4-imidazolyl)thiazole Dihydrochloride Trihydrate

### Method A

To a 50 ml, 3-neck round bottom flask, equipped with reflux condenser, thermometer and mechanical stirrer was added pentylamine (15.0 g, 172 mmol) and 2-guanidino-4-(2-methyl-4-imidazolyl)thiazole dihydrochloride (10.0 g, 23.8 mmol). The thick slurry was heated to reflux (104°C) under nitrogen, and at approximately 90°C the reaction mixture became homogeneous. The reaction was heated at reflux for 19 hours. TLC and HPLC assays indicated that the reaction was complete. The thick brown syrup was cooled to 50°C and then 130 ml of acetone was added followed by 12 ml. of concentrated HCl. Solids started to precipitate. The slurry was cooled to room temperature and granulated for 1 hour. The off-white solids were filtered, washed with acetone, and then air-dried at ambient temperature. The crude dried solid weighed 6.93 g (48.8% yield).

The crude product was dissolved in 80 ml of warm (45°C) acetone/water (1:1) and then treated with 1.0 gram of activated carbon. The carbon-treated solution was filtered through a funnel precoated with diatomaceous earth, and then the filter pad was washed with 11 ml of acetone/water (1:1). The filtrate was transferred to a clean flask and cooled in an ice bath. Concentrated HCl (32 ml) was added slowly. Solids precipitated after approximately 20% of the concentrated acid was added. Once the acid addition was completed, the solids were granulated for 1.0 hour at ambient temperature. The white solid was filtered, washed with acetone and then air-dried to afford crystalline trihydrate dihydrochloride salt (5.85 g, 41.5% overall yield). Microscopic examination under a polarizing microscope showed needle-like crystals. Differential scanning showed a major endotherm at 107-109°C., unchanged on vacuum drying without heat.

Anal. Calcd. for C₁₃H₂₀N₆S.(HCl)₂.(H₂O)₃: C, 37.22; H, 6.74; N, 20.04; S, 7.64; Cl⁻, 16.90; H₂O, 12.87.

Found: C, 36.97; H, 6.57; N, 19.89; S, 7.82; Cl⁻, 16.83; H₂O, 13.32.

On storage at temperatures above 50°C the product loses water. For example, after one week at 70°C, the water level was reduced to 5.69%. On reequilibration at room temperature and 84% relative humidity for two weeks, the trihydrate reformed.

Anal. Found: C, 37.02; H, 6.66; N, 19.83; H₂O, 13.45.

On vacuum drying at 65°C to constant weight, microscopic examination indicated the resulting anhydrous product to be amorphous. Differential scanning calorimetry no longer showed the 107-109°C endotherm.

HPLC analyses were conducted on a microBONDAPAK C18 column (7.8 mm ID x 30 cm) using UV detector (254 nm). An eluant containing aqueous buffer and methanol (1:1) was used at 1 ml/min. The buffer contained 0.05M KH₂PO₄, 0.01M Na hexanesulfonate and 0.1% triethylamine which was adjusted to pH 3.0 with phosphoric acid.

TLC analyses were conducted on Merck Pre-Coated silica gel plates (60F-254) using a methanol/water/diethylamine eluant (20:4:1).

Variation in stoichiometry, reaction time and temperature led to the following results:

| Variables | | | | Overall Yields | |
|---|---|---|---|---|---|
| Molar Equiv. Amine | Acid (Molar Equiv.) | Temp. (°C) | Time (hrs.) | Crude (%) | Recryst. (%) |
| 5.1 | HCl (2.0) | 104 | 19 | 48 | -- |
| 10.0 | HCl (2.0) | 102 | 24 | 38 | 32 |
| 10.0 | HCl (2.0) | 102-107 | 19 | 48 | 43 |
| 17.6 | HCl (2.0) | 103 | 17.5 | 43 | -- |
| 13.4 | HOAc^{a}(1.0) | 104 | 32 | 60 | -- |
| 13.4 | PTS^{b}(1.0) | 104 | 18 | 61^{c} | -- |

| | | | | | |
|---|---|---|---|---|---|
| ^{a} acetic acid | | | | | |
| ^{b} p-toluenesulfonic acid | | | | | |
| ^{c} by HPLC assay | | | | | |

### Method B

The amorphous dihydrochloride of 2-(1-pentyl-3-guanidino)-4-(2-methyl-4-imidazolyl)thiazole was prepared according to Example 5 US-A-4,560,690. Thus 8.1 g of the dihydrobromide salt was dissolved in 150 ml of H₂O at 50°C. Na₂CO₃.H₂O (8.86 g.) in 80 ml H₂O was added over 1 hour with stirring. After stirring for an additional 0.5 hour at ambient temperature, the base was recovered by filtration and incompletely dried for 48 hours in vacuo. The entire batch was taken into 200 ml acetone, clarified by filtration and the filtrate acidified with 3.4 ml 12N HCl and diluted with 100 ml fresh acetone. Recovery by filtration and drying at 60° in vacuo for 24 hours gave 6.3 g of amorphous dihydrochloride correctly analyzing for C₁₃H₂₀N₆S.(HCl)₂.0.5H₂O: Calcd.: C, 41.71; H, 6.19; N, 22.45. Found: C, 41.90, 41.60; H, 6.20, 6.23; N, 22.55, 22.48. This product (3.01 g.) was dissolved in 75 ml H₂O with stirring. Activated carbon, 0.3 g, was added and the mixture stirred for 15 minutes and then filtered. Concentrated HCl (25 ml.) was added to the filtrate. Crystallization began within a few minutes. After granulating for 1 hour, crystalline title product was recovered by filtration, with 4 ml H₂O wash, and air dried for 18 hours, 2.9 g; water content 12.6%; physical properties as product of Method A. The product was optionally washed with a samll amount of acetone, or repulped in 60 ml of CH₃CN for 2 hours and refiltered, in order to facilitate drying.

The product was optionally recrystallized by dissolving 3.4 g of the trihydrate in 34 ml of H₂O by warming to 50°C CH₃CN (180 ml.) was added, maintaining 50°C. The mixture was cooled slowly with stirring to 20°C. Crystallization began at 32°C. The product was recovered as the trihydrate (water content 12.95%) having unchanged physical properties.

### EXAMPLE 2

### N-(Pentylamidino)thiourea

To a one liter, 3-necked round bottom flask which was equipped with a mechanical stirrer, thermometer, and reflux condenser were added absolute ethanol (180 ml.) and pentylamine (100.0 g, 1.15 mol). The amine solution was cooled in an ice bath while glacial acetic acid (68.9 g, 1.15 mol) was slowly added. Amidinothiourea (90.37 g, 0.76 mol) was added and then the mixture was heated to reflux (85°C). After 24 hours at reflux, HPLC analysis of the reaction solution indicated that title product (72.6 area %) had formed and that residual amidinothiourea (8.5 area %) remained.

Hot water (750 ml) was added to the amber solution. After filtration, the solution was slowly cooled to room temperature. A thick, white slurry formed and was granulated for 30 minutes at ambient temperature. The solid was filtered, washed with water (50 ml) followed by hexane (150 ml) and then air dried. Crude title product was obtained in approximately 50% yield as the partial acetate salt.

The crude product was dissolved in ethyl acetate (500 ml) and then washed with 5% NaHCO₃ solution (2 x 100 ml). The ethyl acetate layer was dried and then heated to reflux. Cyclohexane (700 ml) was added and the solution was allowed to cool to room temperature. The solids were filtered, washed with cyclohexane and then air dried. Purified title product was isolated as a sharp melting solid; m.p. 102-104°C.

The reaction was repeated, varying the proportion of reagents, acid catalyst, solvent reaction time and temperature with the following results:

| Variables | | | | | Yield | |
|---|---|---|---|---|---|---|
| Molar Equiv. Amine | Acid (Molar Equiv.) | Solvent | Time (hr.) | Temp. (°C.) | HPLC Assay | Isolated |
| 1.5 | HOAc^{a} (1.5) | EtOH^{b} | 67 | 85 | 73 | 48 |
| 1.4 | HOAc^{a} (2.0) | EtOH^{b} | 60 | 87 | 67 | 58 |
| 1.0 | HOAc^{a} (1.0) | IPO^{c} | 49 | 70 | 51 | -- |
| 5.0 | NH₄Cl (2.0) | IPO^{c} | 55 | 50 | 34 | -- |

| | | | | | | |
|---|---|---|---|---|---|---|
| ^{a} acetic acid | | | | | | |
| ^{b} ethanol | | | | | | |
| ^{c} isopropanol | | | | | | |

### EXAMPLE 3

### Capsule Containing 100 mgA of 2-(1-pentyl-3-guanidino)-4-(2-methyl-4-imidazolyl)thiazole

### Method A

The following materials were thoroughly blended in the indicated proportions by weight:

| | |
|---|---|
| 2-(1-pentyl-3-guanidino)-4-(2-methyl-4-imidazolyl)thiazole dihydrochloride trihydrate | 144.9* |
| lactose, anhydrous | 173.8 |
| starch 1500 | 173.8 |
| magnesium stearate | 7.5 |

| | |
|---|---|
| *144.9 mg of dihydrochloride trihydrate is equivalent to 100 mg of anhydrous free base, i.e., 100 mgA (100 mg of activity). | |

Hard gelatin capsules of suitable size were filled with 500 mg each of this blend to form containing 100mgA/capsule. These capsules showed good chemical and physical stability after storage under challenge conditions (96% drug released in 15 minutes in water using standard dissolution methods).

Capsules containing 50 mgA are analogously prepared using 500 mg of a blend composed of 72.5 parts by weight of dihydrochloride trihydrate, 211.2 parts by weight of each of anhydrous lactose and starch 1500, and 5.1 parts by weight of magnesium stearate.

## Claims (Claims for the following Contracting State(s): AT, BE, CH, DE, FR, GB, IT, LI, LU, NL, SE)

1. A process for the preparation of a compound of the formula 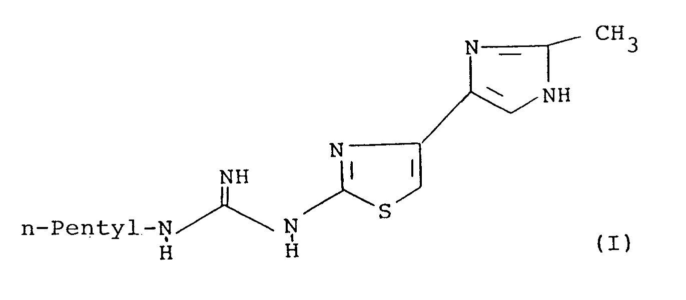 or a pharmaceutically acceptable acid addition salt thereof, which comprises heating a compound of the formula 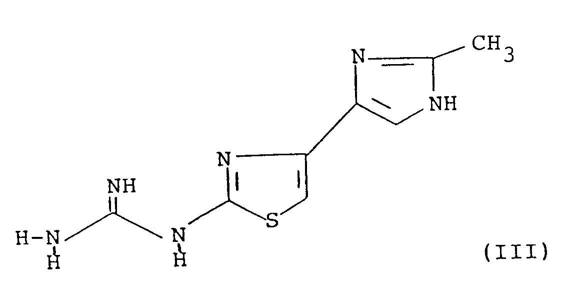 either in acid addition salt form, or in the presence of an acid catalyst, with at least one molar equivalent of n-pentylamine either neat or in a reaction-inert solvent.

2. A process of claim 1 which employs the dihydrochloride salt of the compound of the formula (III) in a molar excess of the amine.

3. A process for the preparation of a compound of the formula 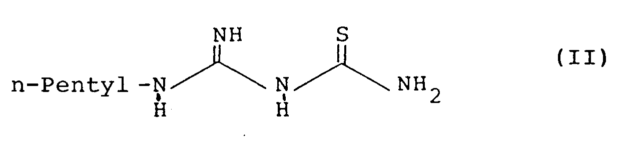 or an acid addition salt thereof, which comprises heating an amidinothiourea of the formula 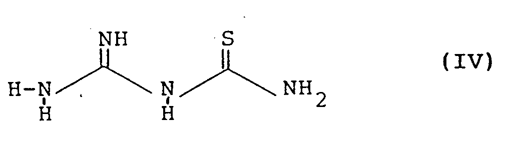 either in acid addition salt form, or in the presence of an acid catalyst, with at least one molar equivalent of n-pentylamine either neat or in a reaction-inert solvent.

4. A process of claim 3 which employs said amidinothiourea and at least one molar equivalent of acetic acid or ammonium chloride as the acid catalyst in a lower alkanol.

5. The process of claim 4 wherein the acid catalyst is acetic acid and the solvent is ethanol.

6. Crystalline 2-(1-pentyl-3-guanidino)-4-(2-methyl-4-imidazolyl)thiazole dihydrochloride trihydrate.

## Claims (Claims for the following Contracting State(s): ES, GR)

1. A process for the preparation of a compound of the formula 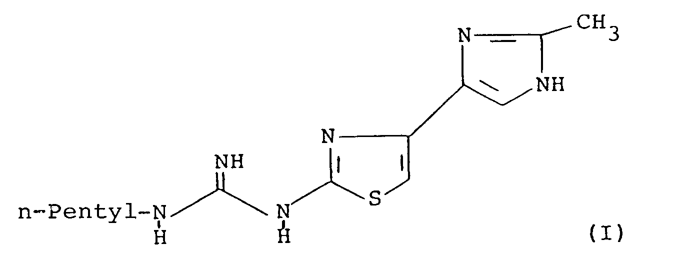 or a pharmaceutically acceptable acid addition salt thereof, which comprises heating a compound of the formula 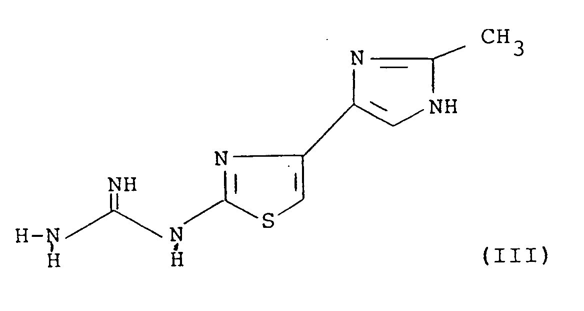 either in acid addition salt form, or in the presence of an acid catalyst, with at least one molar equivalent of n-pentylamine either neat or in a reaction-inert solvent.

2. A process of claim 1 which employs the dihydrochloride salt of the compound of the formula (III) in a molar excess of the amine.

3. A process for the preparation of a compound of the formula 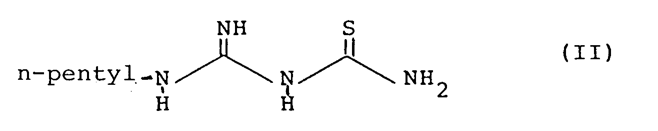 or an acid addition salt thereof, which comprises heating an amidinothiourea of the formula 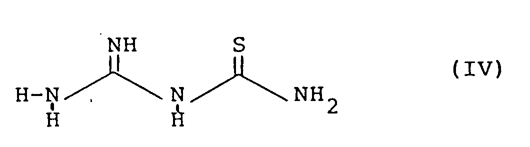 either in acid addition salt form, or in the presence of an acid catalyst, with at least one molar equivalent of n-pentylamine either neat or in a reaction inert solvent.

4. A process of claim 3 which employs said amidinothiourea and at least one molar equivalent of acetic acid or ammonium chloride as the acid catalyst in a lower alkanol.

5. The process of claim 4 wherein the acid catalyst is acetic acid and the solvent is ethanol.

6. A process for the preparation of 2-(1-pentyl-3-guanidino)-4-(2-methyl-4-imidazolyl)thiazole dihydrochloride trihydrate which comprises crystallization from dilute aqueous hydrochloric acid optionally diluted with a water miscible organic solvent.

7. The process of claim 6 wherein the aqueous acid is diluted with acetonitrile.

## Patentansprüche (Patentansprüche für folgende(n) Vertragsstaat(en): AT, BE, CH, DE, FR, GB, IT, LI, LU, NL, SE)

1. Verfahren zum Herstellen einer Verbindung mit der Formel 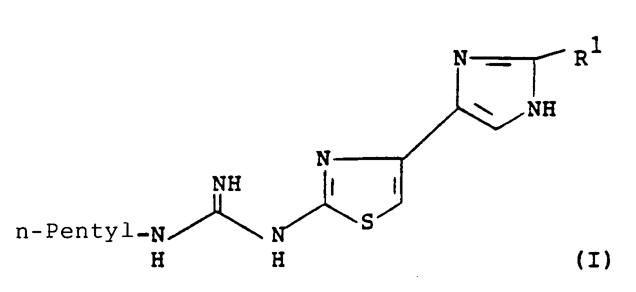 oder eines pharmazeutisch annehmbaren Säure-Additionssalzes davon, das umfaßt das Erhitzen einer Verbindung mit der Formel 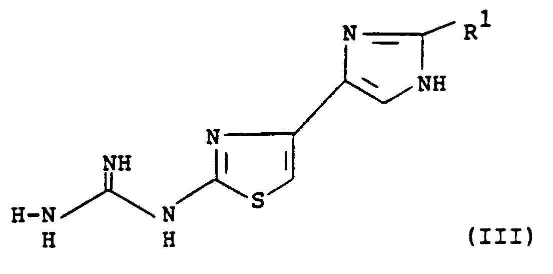 entweder in Form des Säure-Additionssalzes oder in Gegenwart eines sauren Katalysators mit mindestens einem Moläquivalent n-Pentylamin entweder unverdünnt oder in einem reaktionsinerten Lösungsmittel.

2. Verfahren nach Anspruch 1, das das Dihydrochlorid-Salz der Verbindung mit der Formel (III) in einem molaren Überschuß des Amins verwendet.

3. Verfahren zum Herstellen einer Verbindung mit der Formel 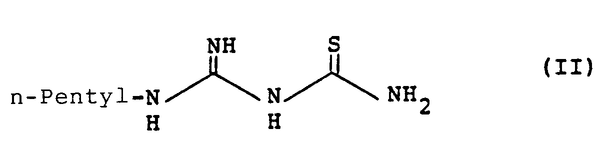 oder eines Säure-Additionssalzes davon, das umfaßt das Erhitzen eines Amidinothioharnstoffes mit der Formel 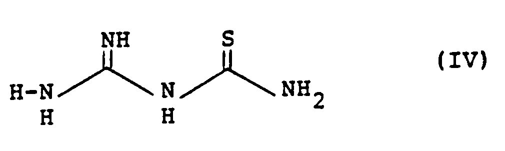 entweder in Form des Säure-Additionssalzes oder in Gegenwart eines sauren Katalysators mit mindestens einem Moläquivalent n-Pentylamin entweder unverdünnt oder in einem reaktionsinerten Lösungsmittel.

4. Verfahren nach Anspruch 3, das den Amidinothioharnstoff und mindestens ein Moläquivalent Essigsäure oder Ammoniumchlorid als den sauren Katalysator in einem niederen Alkanol verwendet.

5. Verfahren nach Anspruch 4, in dem der saure Katalysator Essigsäure ist und das Lösungsmittel Ethanol ist.

6. Kristallines 2-(1-Pentyl-3-guanidino)-4-(2-methyl-4-imidazolyl)thiazol-Dihydrochlorid-Trihydrat.

## Patentansprüche (Patentansprüche für folgende(n) Vertragsstaat(en): ES, GR)

1. Verfahren zum Herstellen einer Verbindung mit der Formel 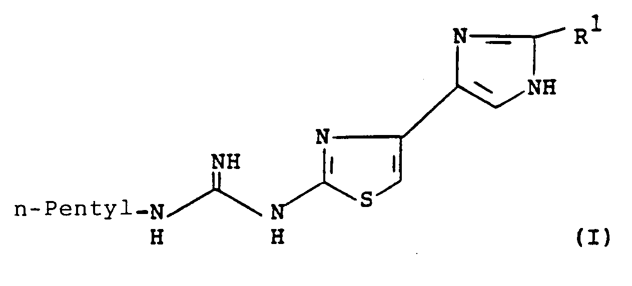 oder eines pharmazeutisch annehmbaren Säure-Additionssalzes davon, das umfaßt das Erhitzen einer Verbindung mit der Formel 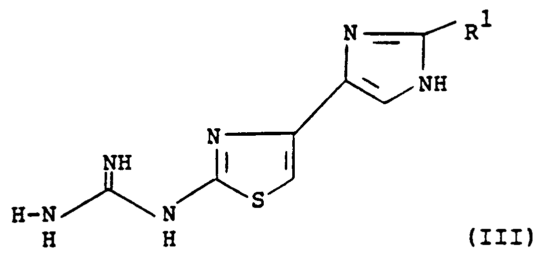 entweder in Form des Säure-Additionssalzes oder in Gegenwart eines sauren Katalysators mit mindestens einem Moläquivalent n-Pentylamin entweder unverdünnt oder in einem reaktionsinerten Lösungsmittel.

2. Verfahren nach Anspruch 1, das das Dihydrochlorid-Salz der Verbindung mit der Formel (III) in einem molaren Überschuß des Amins verwendet.

3. Verfahren zum Herstellen einer Verbindung mit der Formel 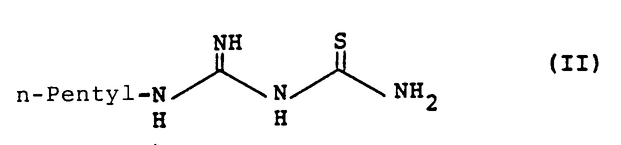 oder eines Säure-Additionssalzes davon, das umfaßt das Erhitzen eines Amidinothioharnstoffes mit der Formel 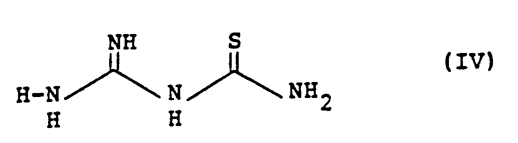 entweder in Form des Säure-Additionssalzes oder in Gegenwart eines sauren Katalysators mit mindestens einem Moläquivalent n-Pentylamin entweder unverdünnt oder in einem reaktionsinerten Lösungsmittel.

4. Verfahren nach Anspruch 3, das den Amidinothioharnstoff und mindestens ein Moläquivalent Essigsäure oder Ammoniumchlorid als den sauren Katalysator in einem niederen Alkanol verwendet.

5. Verfahren nach Anspruch 4, in dem der saure Katalysator Essigsäure ist und das Lösungsmittel Ethanol ist.

6. Verfahren zum Herstellen von 2-[1-Pentyl-3-guanidino]-4-(2-methyl-4-imidazolyl)thiazol-Dihydrochlorid-Trihydrat, das umfaßt das Kristallisieren aus verdünnter wäßriger Salzsäure, die fakultativ mit einem mit Wasser mischbaren organischen Solvens verdünnt ist.

7. Verfahren nach Anspruch 6, in dem die wäßrige Säure mit Acetonitril verdünnt ist.

## Revendications (Revendications pour l'(les) Etat(s) contractant(s) suivant(s): AT, BE, CH, DE, FR, GB, IT, LI, LU, NL, SE)

1. Procédé de préparation d'un composé de formule 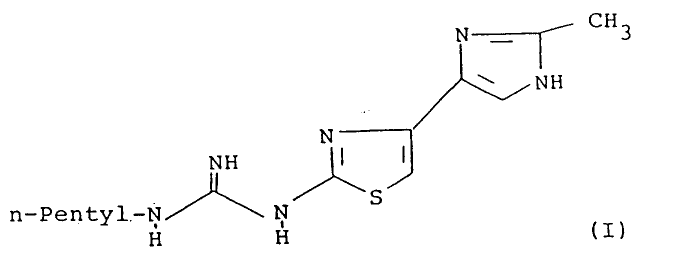 ou d'un de ses sels d'addition d'acide pharmaceutiquement acceptables, qui comprend le chauffage d'un composé de formule 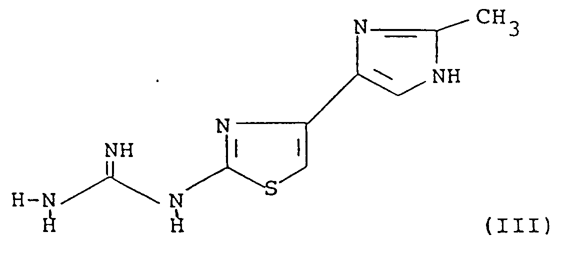 sous forme d'un sel d'addition d'acide, ou bien en présence d'un catalyseur acide, avec au moins un équivalent molaire de n-pentylamine à l'état pur ou dans un solvant inerte vis-à-vis de la réaction.

2. Procédé suivant la revendication 1, qui utilise le dichlorhydrate du composé de formule (III) dans un excès molaire de l'amine.

3. Procédé de préparation d'un composé de formule 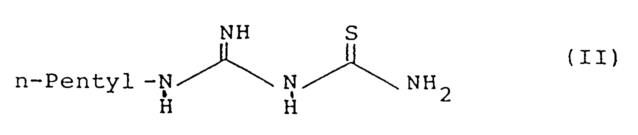 ou d'un de ses sels d'addition d'acide, qui comprend le chauffage d'une amidinothiourée de formule 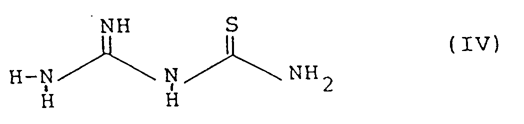 sous forme d'un sel d'addition d'acide, ou bien en présence d'un catalyseur acide, avec au moins un équivalent molaire de n-pentylamine à l'état pur ou dans un solvant inerte vis-à-vis de la réaction.

4. Procédé suivant la revendication 3, qui utilise l'amidinothiourée et au moins un équivalent molaire d'acide acétique ou de chlorure d'ammonium comme catalyseur acide dans un alcanol inférieur.

5. Procédé suivant la revendication 4, dans lequel le catalyseur acide est l'acide acétique et le solvant est l'éthanol.

6. Trihydrate de dichlorhydrate de 2-(1-pentyl-3-guanidino)-4-(2-méthyl-4-imidazolyl)thiazole cristallin.

## Revendications (Revendications pour l'(les) Etat(s) contractant(s) suivant(s): ES, GR)

1. Procédé de préparation d'un composé de formule 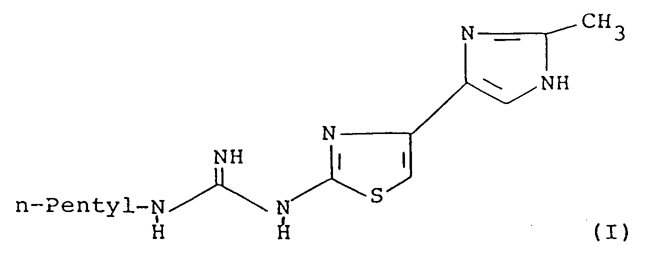 ou d'un de ses sels d'addition d'acide pharmaceutiquement acceptables, qui comprend le chauffage d'un composé de formule 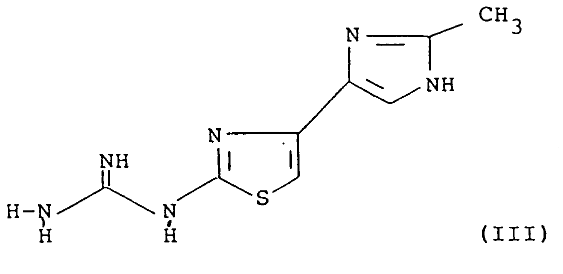 sous forme d'un sel d'addition d'acide, ou bien en présence d'un catalyseur acide, avec au moins un équivalent molaire de n-pentylamine à l'état pur ou dans un solvant inerte vis-à-vis de la réaction.

2. Procédé suivant la revendication 1, qui utilise le dichlorhydrate du composé de formule (III) dans un excès molaire de l'amine.

3. Procédé de préparation d'un composé de formule 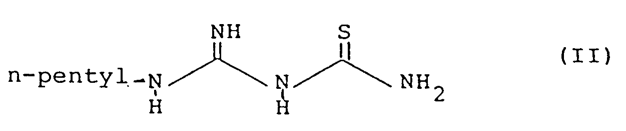 ou d'un de ses sels d'addition d'acide, qui comprend le chauffage d'une amidinothiourée de formule 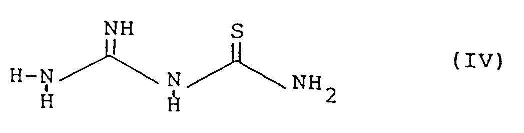 sous forme d'un sel d'addition d'acide, ou bien en présence d'un catalyseur acide, avec au moins un équivalent molaire de n-pentylamine à l'état pur ou dans un solvant inerte vis-à-vis de la réaction.

4. Procédé suivant la revendication 3, qui utilise l'amidinothiourée et au moins un équivalent molaire d'acide acétique ou de chlorure d'ammonium comme catalyseur acide dans un alcanol inférieur.

5. Procédé suivant la revendication 4, dans lequel le catalyseur acide est l'acide acétique et le solvant est l'éthanol.

6. Procédé de préparation de trihydrate de dichlorhydrate de 2-(1-pentyl-3-guanidino)-4-(2-méthyl-4-imidazolyl)thiazole, qui comprend la cristallisation dans une solution aqueuse d'acide chlorhydrique diluée, facultativement à l'état dilué avec un solvant organique miscible à l'eau.

7. Procédé suivant la revendication 6, dans lequel la solution aqueuse d'acide est diluée avec de l'acétonitrile.
